# EUROPÄISCHE PATENTSCHRIFT

(11) **EP 2 931 358 B1**
(45) Veröffentlichungstag und Bekanntmachung des Hinweises auf die Patenterteilung: **15.02.2017**
(21) Anmeldenummer: 13818994.9
(22) Anmeldetag: 12.12.2013
(51) Int. Cl.: B29L 23/00, A61M 39/10, B29C 45/00, F16L 19/00, F16L 21/08

(54) **ANSCHLUSSSYSTEM MIT GEWINDEMUTTER**
CONNECTING SYSTEM WITH THREADED NUT
SYSTÈME DE BRANCHEMENT AVEC ÉCROU FILETÉ

(30) Priorität: 13.12.2012 DE 102012112212
(43) Veröffentlichungstag der Anmeldung: 21.10.2015
(73) Patentinhaber: Fresenius Kabi Deutschland GmbH, 61352 Bad Homburg (DE)
(72) Erfinder: HOPF, Michael, 90513 Zirndorf (DE); KASSAI, Norbert, 90522 Oberasbach (DE); HOPF, Alexander, 90491 Nürnberg (DE); HOPF, Hans-Jürgen, 90513 Zirndorf (DE)
(74) Vertreter: Wittmann, Ernst-Ulrich
(86) Internationale Anmeldenummer: PCT/EP2013/076428
(87) Internationale Veröffentlichungsnummer: WO 2014/090958

(56) Entgegenhaltungen:
- WO-A1-2013/123028
- DE-A1-102009 044 319
- DE-U1- 20 302 788

## Beschreibung

Die vorliegende Erfindung betrifft ein Anschlusssystem für Fluidverbindungen, wie sie insbesondere in der Medizin und Medizintechnik eingesetzt werden.

Anschlusssysteme für Fluidverbindungen sind im Stand der Technik bekannt und werden zum Beispiel in der Medizin und Medizintechnik eingesetzt. Hierbei werden die Anschlusssysteme im Infusionsbereich, dem Bereich der künstlichen Ernährung, bei Spritzen und Injektionsbesteck, bei der Transfusion und für die Zu- bzw. Überleitung von verschiedenen Durchflussmedien und als sogenanntes "Injektions-Equipment" für die medizinische und pharmazeutische Ausrüstung verwendet, welche unter anderem auch aus mehreren Komponenten bestehen.

In der Medizin ist u.a. die absolute Sicherheit in der Bedienung von Spritzen, Infusionsschläuchen, Kanülen etc. oberstes Gebot. Daher hat sich in der Medizintechnik das Luer- bzw. Luer-Lock-Prinzip gegenüber früher gebräuchlichen Verbindungssystemen durchgesetzt. Luer-Lock ist ein genormtes Verbindungssystem für Kanülen, Spritzen und Infusions-Schläuche im medizinischen Bereich.

Die Dichtung wird hierbei durch eine kegelförmige Konstruktion der Verbindungsteile (des sogenannten Luer-Konus) erreicht. Dabei wird der Innenkegel der einen Verbindungsseite auch als "weiblich" bezeichnet, der Außenkegel der Gegenseite als "männlich". Der Konus weist eine genormte Steigung von 6 % auf.

Wenn der Konus zur Sicherung bzw. Verriegelung der Verbindung gegen versehentliches Lösen mit einer Gewindemutter bzw. Überwurfmutter mit Gewinde erweitert ist, bezeichnet man das System als Luer-Lock. Die einfachere Version ohne Schraubgewinde an der Spritzendüse wird als Luer-Ansatz, Luer-Steck oder auch "Luer-Slip" bezeichnet.

Das Luer-Lock-System garantiert die Kompatibilität zwischen verschiedenen Herstellern und ist international anerkannt. Weltweit hat sich die Luer-Lock-Verbindung für reversible Verbindungen von Spritzen, Kanülen, Infusionsschläuchen, Spinalnadeln und dergleichen durchgesetzt. Dies stellt einen großen Vorteil, insbesondere im Rahmen internationaler Hilfs- und Katastropheneinsätze, dar.

Gleichzeitig liegt in der Universalität des Systems auch ein dramatischer Nachteil und damit verbundene Gefahren. Die Luer-Lock-Verbindung birgt durch seine universelle Standardisierung gefährliche Verwechslungsmöglichkeiten in sich, da das System kompatibel für vaskuläre (Venen und Arterien) und spinale bzw. peridurale (Rückenmark) Anwendungen, teilweise auch für enterale (z. B. Ernährungssonden) und respiratorische (Atemwege) Anwendungen ist. So verhindert die Verbindungstechnik grundsätzlich nicht, dass z. B. ein venös zu injizierendes Medikament fälschlicherweise spinal (in die Rückenmarkflüssigkeit) appliziert wird. Leider liegen mehrere Fälle solcher Verwechslungen mit tödlichem Ausgang vor.

Darüber hinaus ist es verfahrenstechnisch relativ aufwendig, die Gewindemutter bzw. Überwurfmuttern - wie sie u.a. im Luer-Lock-System verwendet werden - herzustellen, da für das innenliegende Gewinde der Mutterbei der Herstellung im Kunststoffspritzguss ein ausdreh- oder ausfaltbares Werkzeug verwendet werden muss. Dies führt zu einer deutlichen Verteuerung der Werkzeugkosten und erhöht den Aufwand bei der Herstellung. Besonders deutlich wird der Nachteil, wenn an einem Bauteil eine Vielzahl solcher Gewinde vorgesehen ist.

Aus DE 203 02 788 U1 ist ein Anschlusssystem für fluiddurchströmte Bauteile in der Medizin und Medizintechnik bekannt, mit Gewindeabschnitten und abschnittsweise radial umlaufenden Durchbrechungen.

Aufgabe der vorliegenden Erfindung ist es, die im Stand der Technik bekannten Nachteile wenigstens teilweise zu überwinden. Dies betrifft zum einen die Erhöhung der Benutzersicherheit, als auch zum anderen die Vereinfachung des Herstellungsprozesses.

Die Aufgabe der vorliegenden Erfindung wird durch ein Anschlusssystem gemäß Anspruch 1 gelöst. Bevorzugte Ausgestaltungen der Erfindung sind Gegenstand der Unteransprüche, insbesondere auch das Verfahren zur Herstellung dieser Anschlusssysteme und deren Verwendung in der Medizin und Medizintechnik.

Das erfindungsgemäße Anschlusssystem für fluiddurchströmte Bauteile in der Medizin und Medizintechnik weist wenigstens einen männlichen Verbindungskonus und ein, wenigstens teilweise diesen Verbindungskonus umschließende, Gewindemutter mit einem Innengewinde (Muttergewinde) auf.

Dabei dient die Gewindemutterinsbesondere zur verliersicheren Verbindung des männlichen Verbindungskonus mit einer weiblichen Aufnahme eines weiteren, fluiddurchströmten Bauteils. Hierbei weist insbesondere die weibliche Aufnahme außenliegende Vorsprünge auf, welche in das Innengewinde der Gewindemutter eingreifen. Durch Drehen des männlichen Verbindungskonus in die weibliche Aufnahme werden die Bauteile fluiddicht und verliersicher verbunden.

Gemäß der vorliegenden Erfindung weist die Gewindemutter im axialen Abschnitt ihres Umfangsmantels abschnittsweise, radial umlaufende Durchbrechungen auf, durch welche im Umfangsmantel Stege bzw. Stegabschnitte gebildet werden. Entsprechend der vorliegenden Erfindung weist die Gewindemutter ferner auf der Innenseite der zuvor beschriebenen Stege die Abschnitte des Innengewindes auf.

Dabei sind als Abschnitte des Innengewindes insbesondere die Gewindeflanken der entsprechenden Gewindegänge zu verstehen, welche im Anschluss an die Durchbrechungen, d.h. auf der Innenseite der Stege bzw. Stegsegmente angeordnet sind. Gemäß der vorliegenden Erfindung erstrecken sich die Flanken des Innengewindes im Wesentlichen parallel zu den Durchbrechungen und sind auch auf diese Bereiche bzw. Abschnitte begrenzt. Dies kann an allen Stegen bzw. Stegelementen entsprechend vorgesehen sein oder auch nur an einigen dieser Stege bzw. Stegabschnitte.

Gemäß der vorliegenden Erfindung wird dabei als Steg ein Strukturmerkmal des Umfangsmantels verstanden, welches wenigstens an einer Seite an die umlaufende Durchbrechung anschließt. Darüberhinaus können diese Stege auch an zwei Durchbrechungen anschließen, wobei dies nicht zwingend notwendig ist.

Im Verständnis der vorliegenden Erfindung sind u.a. die Durchbrechungen dem Herstellungsverfahren geschuldet, bei welchem Teile des Werkzeugs durch den äußeren Mantel der Gewindemutter hindurchragen und hierdurch die Form zur Bindung des Innengewindes auf der Innenseite der Gewindemutter bzw. Überwurfmutter im Spritzgussverfahren bereitstellen.

Das so hergestellte Innengewinde einer Gewindemutter hat damit den Vorteil, dass für dessen Herstellung kein innerhalb des Gewindes angeordnetes Werkzeug bzw. Werkzeugabschnitt vorgesehen werden muss. Durch einfaches lineares Schließen und Öffnen des Spritzgusswerkzeuges inkl. des ggf. notwendigen Schieber wird somit eine Form für ein innenliegendes Gewinde bereitgestellt, mit welchem die innenliegenden Gewindegänge der Gewindemutter erzeugt werden können. Diese Art der Herstellung hat den Vorteil, dass keine drehbeweglichen oder ausfaltbaren Werkzeugbestandteile zur Formbildung des Innengewindes benutzt werden müssen und sich somit auch die Zykluszeiten im Spritzgussverfahren reduzieren lassen. Ferner dient es zur Gewichtsreduzierung und Materialeinsparung bei der Herstellung des Produktes.

Gemäß einer weiteren besonders bevorzugten Ausführungsform der vorliegenden Erfindung ist das Innengewinde der Gewindemutter r zwei- oder dreigängig ausgeführt. Dabei ist auch zu berücksichtigen, dass gemäß einer weiteren besonders bevorzugten Ausführungsform pro Gewindegang wenigstens zwei Durchbrechungen vorgesehen sind, welche beispielsweise gegenüberliegend am Umfang der Gewindemutter angeordnet sind.

Bei der Verwendung von dreigängigen Gewinden sind die Durchbrechungen je um 120 Grad von einander versetzt angeordnet, wobei pro Ausrichtung und Gewindeeingang wenigstens einer bzw. zwei insbesondere drei in axialer Richtung übereinander angeordneten Durchbrechungen vorgesehen sind, an deren Innenseite jeweils ein entsprechender Abschnitt des Innengewindes angeordnet ist.

Gemäß einer weiteren besonders bevorzugten Ausführungsform der vorliegenden Erfindung erstrecken sich die abschnittsweise radial umlaufenden Durchbrechungen, welche vorzugsweise an der Innenseite an Abschnitte des Innengewindes angrenzen, über ein Drittel, insbesondere über wenigstens zwei Drittel des Umfangs, wobei hier von einer Summe der entsprechenden Innengewindestrukturen pro Steighöhe, d.h. pro 360 Grad gerechnet wird. Dabei ist es auch von Vorteil, dass entweder über die Zahl der Gewindegänge oder die Zahl an Umläufen des Gewindegangs die gewünschte Festigkeit bzw. Beständigkeit der Schraubverbindung beeinflussbar ist. So nimmt mit der Zahl der Gewindegänge nicht nur die "Griffigkeit" des Gewindes zu, sondern auch die Stabilität und Festigkeit der Verbindung bei gleichem sonstigen Aufbau. Dabei ist als Griffigkeit die Art der Gewindeausgestaltung zu verstehen, welche es dem Benutzer vereinfacht, das Außengewinde des weiblichen Gegenstücks mit der Gewindemutter zu verbinden. Diese steigt mit Zahl der Gänge, da die mögliche Zahl der Aufnahmestellen für das Gegenstück in der Gewindemutterzunimmt.

Vorzugsweise sind die entsprechenden Innengewindeabschnitte, welche bei zwei und insbesondere bei dreigängigen Gewinden vorgesehen sind, axial entsprechend der Steighöhe des verwendeten Gewindegangs hintereinander angeordnet.

Gemäß einer weiteren besonders bevorzugten Ausführungsform der vorliegenden Erfindung sind neben den sich im Umfangsmantel erstreckenden Stegen Verstärkungsstreben vorgesehen, mit welchen insbesondere die Stege im Bereich der Durchbrechungen abgestützt werden. Dabei ist zu berücksichtigen, dass die Stege, welche vorzugsweise auf der Innenseite einen Gewindeabschnitt aufweisen, relativ dünn ausgeführt sein können und insbesondere beim Einsatz von zweigängigen Verbindungssystemen relativ große Kräfte auf den Gewindeabschnitt wirken. Um diese Kräfte sicher aufnehmen zu können und ein Durchbrechen der Stege und damit der Gewindeabschnitte wenigstens teilweise zu vermeiden, werden gemäß dieser besonderen Ausführungsform die Verstärkungsstreben im Bereich zwischen den Anbindungspunkten zum Umfangsmantel angeordnet. Hierdurch können insbesondere die beim Einschrauben eines Gegenstücks wirkenden Kräfte besser aufgenommen und abgeleitet werden, ohne dass es zum Bruch des Bauteils kommen muss.

Ein möglicher Anbindungspunkt für die Verstärkungsstreben kann beispielsweise der mittlere Abstand zu dem in radialer Richtung angrenzenden Umfangsmantel sein, wobei sich die Verstärkungsstreben vorzugsweise achsparallel zu dem im Inneren der Gewindemutter angeordneten Verbindungskonus erstrecken. Alternativ hierzu können die Verbindungsstreben auch in einem stumpfen Winkel zu den Stegen angeordnet werden, wobei dieser Winkel vorzugsweise zwischen 75° und 90° liegt.

Gemäß einer weiteren, besonders bevorzugten Ausführungsform der vorliegenden Erfindung wird das vorliegende Anschlusssystem nicht mit den Maßen des standardisierten Luer-Systems versehen, sondern durch Abwandlung der entsprechenden Maße mit einem Verwechslungsschutz ausgestattet. Dies stellt sicher, dass das vorliegende Anschlusssystem durch unterschiedliche Ausführungsgrößen mit den beispielsweise in der Medizin gängigerweise verwendeten Luer-System inkompatibel ist. Dadurch wird insbesondere sichergestellt, dass bei Verwendung des vorliegenden Anschlusssystems, beispielsweise im Bereich der künstlichen Ernährung, eine Verwechslung mit Anschlusssystemen, wie sie beispielsweise in Bereich der Infusions- oder Spritzenbestecke verwendet werden, ausgeschlossen werden kann. Hierzu weist der Außendurchmesser des Innengewindes insbesondere einen Durchmesser auf, der größer als 7,83 mm ist und vorzugsweise größer als 7,83 mm und kleiner als 20 mm ist. Gemäß einer weiteren besonders bevorzugten Ausführungsform ist der Außendurchmesser des Innengewindes größer als 8 mm und kleiner als 10 mm und liegt insbesondere bei ungefähr 9 mm.

Neben dem Außendurchmesser kann gemäß einer weiteren besonders bevorzugten Ausführungsform die Steighöhe des Innengewindes von dem im Luer-System verwendeten Maß abgewandelt werden und ist gemäß der vorliegenden Erfindung vorzugsweise größer als 1,3 mm, des weiteren größer als 1,3 mm und kleiner als 5 mm. Gemäß einer weiteren besonders bevorzugten Ausführungsform ist die Steighöhe insbesondere größer als 1,5 mm und kleiner als 3 mm und besonders bevorzugt liegt diese zwischen 1,8 mm und 2,5 mm.

Entsprechend einer weiteren besonders bevorzugten Ausführungsform der vorliegenden Erfindung werden wenigstens Teile des Anschlusssystems aus einem Material hergestellt, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, Kombinationen hiervon und dergleichen umfasst.

Gemäß einer weiteren, besonders bevorzugten Ausführungsform der vorliegenden Erfindung ist die Gewindemutter mit dem Verbindungskonus drehfest verbunden und insbesondere mit dem Verbindungskonus einstückig ausgebildet.

Gemäß einer hierzu alternativen Ausführungsform weist der Verbindungskonus eine radial umlaufende Nut auf, in welcher das Muttergewinde als Überwurfmutter ausgeführt und drehbeweglich angeordnet ist. Hierzu werden der Verbindungskonus und diese Überwurfmutter in getrennten Arbeitsschritten hergestellt und anschließend mechanisch miteinander verbunden.

Die vorliegende Erfindung wird auch durch ein Spritzgussverfahren zur Herstellung eines Anschlusssystems wie zuvor beschrieben erfüllt, wobei dieses Verfahren nur die Herstellung eines Verbindungskonus mit drehfest verbundener Gewindemutter oder der Überwurfmutter - als einzelnes Bauteil - umfasst.

Hierzu umfasst das Verfahren das Schließen der Werkzeugform zur Bildung wenigstens des Verbindungskonus und/oder der Gewindemutter bzw.

Überwurfmutter, das anschließende Einfahren eines Schiebers zur Bildung von Strömungswegen und Freiräumen innerhalb des Bauteils, das Anspritzen des Kunststoffes, das Abkühlen des Kunststoffes in der Werkzeugform und das Ausfahren der Schieber und Öffnen der Werkzeugform zur Entnahme (Entformen) der Kunststoffbauteile.

Mit diesem Verfahren wird deutlich, dass entgegen den sonst üblicherweise zu verwendenden Schiebern zur Bildung der Strömungswege bzw. insbesondere drehbaren Schiebern zur Bildung der Innengewinde der Gewindemutter bzw. Überwurfmutter drehfeste Schieber verwendbar sind, da die Bildung der Innengewinde über die Außenform der Werkzeuge bereitgestellt wird. Dies hat den besonderes bevorzugten Vorteil, dass zusätzliche Drehbewegungen innerhalb des Werkzeuges insbesondere der Schieber vermieden werden können, so dass die Werkzeugform kostengünstig, insbesondere bei der Herstellung einer Vielzahl von entsprechenden Innengewinden an unterschiedlichen Positionen eines Anschlusssystems, beispielsweise bei der Verwendung von Mehrwegehähnen besonders kostengünstig, realisiert werden kann. Dabei ergibt sich die kostengünstiger Herstellung durch die Verkürzung der Zykluszeiten im Spritzgussverfahren. So liegt zum Beispiel die typische Zykluszeit bei der Herstellung von Überwurfmuttern bei ca. 21 bis 22 Sekunden im Spritzgussverfahren, wobei hier vor und nach dem Anspritzen die entsprechenden Gewindeschieber eingedreht bzw. ausgedreht werden müssen. Im Vergleich hierzu kann bei der Herstellung der erfindungsgemäßen Gewindemutter bzw. Überwurfmutter die Zykluszeit um ca. 4 bis 5 Sekunden reduziert werden. Darüber reduzieren sich durch den vereinfachten Aufbau der Werkzeugform die entsprechenden damit verbundenen Kosten um bis zu 40 %. So kann zum Beispiel bei einfachen Werkzeugen auf Zusätze wie Kernzugeinrichtungen verzichtet werden.

Die vorliegende Erfindung umfasst neben dem Anschlusssystem und dessen Verfahren zur Herstellung auch die Verwendung des Anschlusssystems für die Herstellung entsprechend fluiddurchströmter Bauteile in der Medizin und der Medizintechnik, wie beispielsweise Infusions- oder Transfusionsschläuche, Y-Verbinder, Mehrwegehähne, Mehrfachverteiler, Injektionsequipment wie Nadeln, Zugänge oder ähnliches und Kombination hiervon.

Die Erfindung wird nachfolgend anhand verschiedener Ausführungsbeispiele bei der Verwendung des Anschlusssystems in Kombination mit Drei-Wege-Hähnen erklärt, wobei darauf hingewiesen wird, dass die Erfindung nicht auf diese Ausführungsbeispiele beschränkt ist. Es liegt vielmehr im Sinne der vorliegenden Erfindung, dass das Anschlusssystem bezüglich der erfindungsgemäßen Verwendung weit verstanden wird und dass insbesondere Abwandlungen wie sie der Fachmann zur Anpassung an entsprechende Bauteile vornehmen würde, auch im Sinn der vorliegenden Erfindung liegen.

Dabei zeigen:
die Figuren 1a bis 1 c einen Drei-Wege-Hahn mit einem erfindungsgemäßen Anschlusssystem;
Figur 2a und 2b den Drei-Wege-Hahn aus Figur 1 jedoch ohne das Küken;
Figur 3a und 3b eine alternative Ausführungsform, bei welcher die Überwurfmutter in einem weiteren Fertigungsschritt drehbeweglich angeordnet wird;
die Figuren 4a bis 4d schematische Darstellungen der Kunststoffspritzwerkzeuge zur Herstellung eines Drei-Wege-Hahns mit Verbindungskonus mit und ohne Gewindemutter und
die Figuren 5a bis 5e eine alternative Ausführungsform der Gewindemutter mit Verstärkungsstegen.

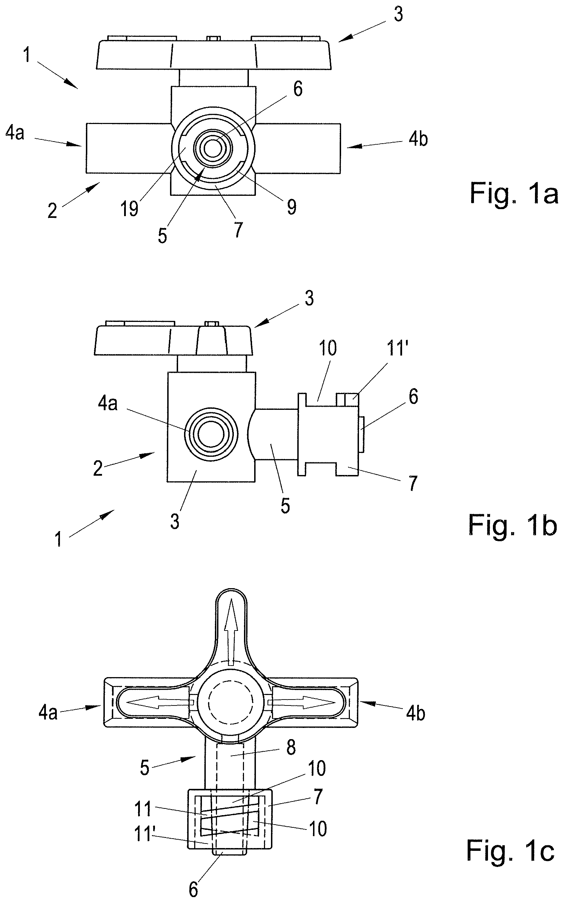

Figur 1 zeigt eine erste Ausführungsform des erfindungsgemäßen Anschlusssystems in Kombination mit einem Drei-Wege-Hahn 1. Neben dem Gehäuse 2 sind das Küken 3 und die Anschlussstellen 4a, 4b und 5 zu erkennen. An dem Anschluss 5 ist der Verbindungskonus 6 mit der Gewindemutter 7 dargestellt, wobei Figur 1a in der Seitenansicht neben dem Umfangsmantel auch das Innengewinde 9 darstellt. Dieses erstreckt sich gemäß der hier dargestellten Ausführungsform je um ca. 135 Grad, so dass sich auf der rechten und linken Seite des Umfangsmantels Freiräume 19 ergeben. In der Draufsicht 1c sind deutlich die Durchbrechungen 10 zu erkennen, welche den Steg 11 bzw. den Stegabschnitt 11' bestimmen. In der Figur 1 b ist der Drei-Wege-Hahn 1 mit dem erfindungsgemäßen Anschlusssystem in einer weiteren Seitenansicht dargestellt, wobei auch hier insbesondere die Durchbrechungen 10 und der Stegabschnitt 11' zu erkennen sind. In der hier dargestellten Ausführungsform ist die Gewindemutter 7 fest mit dem Verbindungskonus 6 bzw. der Anschlussstelle 5 verbunden. Zur Verbindung mit einer weiblichen Aufnahme wird diese in die Mutter 7 eingedreht, so dass der männliche Verbindungskonus aufgenommen und fluiddicht mit der weiblichen Aufnahme verbunden wird (nicht dargestellt).

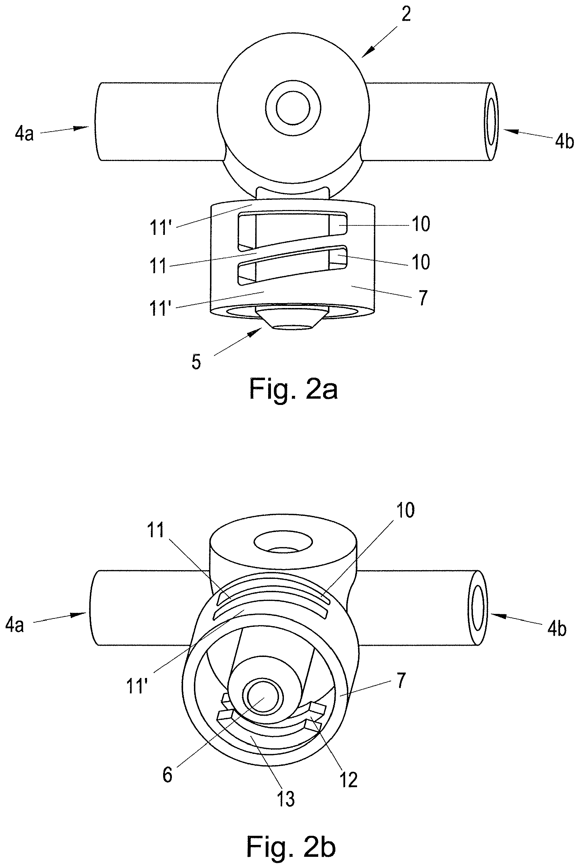

In der Figur 2a und 2b ist das Gehäuse 2 mit der erfindungsgemäßen Gewindemutter 7 in perspektivischer Darstellung wiedergegeben, wobei aus darstellungstechnischen Gründen das Küken nicht abgebildet ist. Auch hier sind neben den Anschlüssen 4a und 4b der Anschluss 5 zu erkennen, an welchen die Gewindemutter 7 angeordnet ist. Diese weist - wie auch bereits in der Figur 1 c zu erkennen ist - die Durchbrechungen 10 und den Steg 11 bzw. Stegabschnitt 11' auf. Figur 2b entspricht dem Bauteil aus Figur 2a, wobei in dieser Darstellung zusätzlich der Verbindungskonus 6 deutlich dargestellt ist. Ferner sind innenliegend die Gewindegänge 12 und 13 zu erkennen, welche sich im Anschluss auf der Innenseite zu den Durchbrechungen 10 erstrecken.

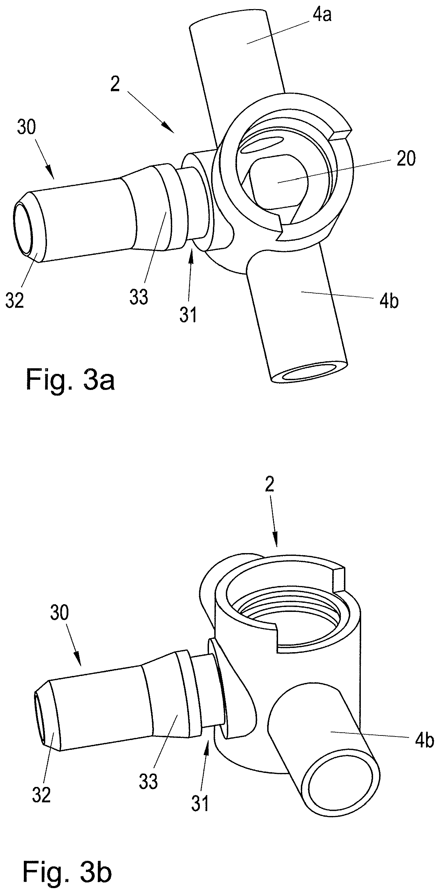

In den Figuren 3a und 3b ist eine alternative Ausführungsform für die Anordnung des erfindungsgemäßen Anschlusssystems wiedergegeben, wobei hier auf den Verbindungskonus 30 eine Überwurfmutter (nicht dargestellt) aufsteckbar ist, welche dann entsprechend in der umlaufenden Nut 31 einrastet. Durch dieses Zusammenführen der beiden Bauteile erhält man eine drehbewegliche Überwurfmutter, welche insbesondere den Vorteil hat, dass der Drei-Wege-Hahn beispielsweise in der Anwendung für die Verbindung des männlichen Verbindungskonus 30 mit einer weiblichen Aufnahme (nicht dargestellt) nicht verdreht werden muss. Besonders deutlich wird hierbei, dass der Verbindungskonus 30 neben der konischen Abstufung 32 am vorderen Ende eine weitere konische Verdickung 33 aufweist, die insbesondere dazu dient, in Verbindung mit der weiblichen Aufnahme eine entsprechende Abdichtung des Verbindungssystems bereitzustellen. Als Überwurfmutter wird hierbei vorzugsweise eine Ausführungsform gewählt, wie diese in Bezug auf die Figuren 1 und 2 dargestellt ist, jedoch ohne den entsprechenden Drei-Wege-Hahn und mit einer zentralen Öffnung auf der Rückseite zur Aufnahme der Anschlussstelle 30 und zum Einrasten in die umlaufende Nut 31.

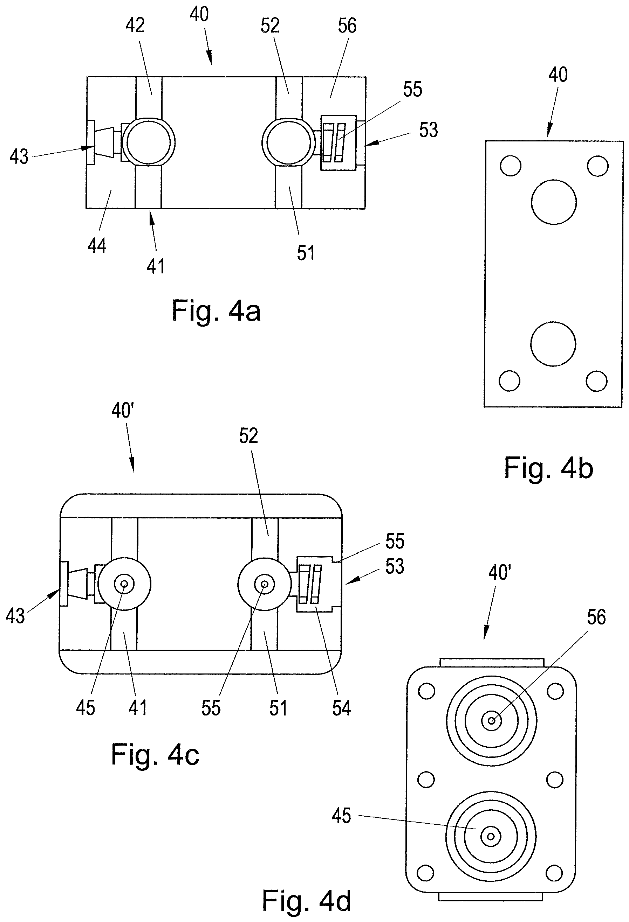

In den Figuren 4a bis 4d sind die beide Werkzeugteile 40 und 40' (4a und 4c: Innenansicht; 4b und 4d: Außenansicht) zur Herstellung des erfindungsgemäßen Anschlusssystems in Kombination mit einem Drei-Wege-Hahn dargestellt, wobei neben den Ausnehmungen für die Fluidanschlüsse 51 und 52 auf der rechten Seite auch der Anschluss 53 zu erkennen ist, an welchen in Verbindung mit dem Verbindungskonus durch die Vorsprünge 55 die Gewindemutter in ihrem Mantel durchbrochen wird und auf der Innenseite entsprechende Gewindegänge erzeugt werden. Hierzu fährt bei der Herstellung in den Freiraum des Anschlusses 53 ein entsprechender Schieber ein, welcher den innenliegenden Gegenpart zur Außenform bildet. Entsprechendes gilt auch für die Fluidanschlüsse 51 und 52, welche keine Überwurfmutter bzw. einen innenliegenden Gewindegang aufweisen. In den Figuren 4c und 4d ist das Gegenstück zu dem Werkzeug aus Fig. 4a und 4b wiedergegeben, wobei neben den entsprechenden Bauteilelementen auch die Anspritzpunkte 45 und 56 zu erkennen sind. Hierzu zeigt die Figur 4d die Anspritzpunkte auf der Rückseite des Werkzeuges.
Entsprechend dem gängigen Verfahren zur Herstellung solcher Spritzgussteile werden im Spritzgussverfahren die beiden Bauteile 4a/4b und 4c/4d zusammengeführt und bilden den Grundkörper für den Drei-Wege-Hahn mit der erfindungsgemäßen Gewindemutter bzw. Überwurfmutter. Durch Einführen entsprechender Schieber (nicht dargestellt) in die Öffnungen 51, 52, 53 bzw. 41, 42, 43 werden die Freiräume für die Fluidwege geschaffen. Mit dieser Spritzgussform kann ferner neben der wie zuvor beschriebenen Mutter mit Innengewinde auch der Drei-Wege-Hahn mit einer fest angeordneten Gewindemutter auf der rechten Seite und einem Drei-Wege-Hahn mit einer umlaufenden Nut 44 zur Aufnahme oder Anordnung einer Überwurfmutter auf der linken Seite erzeugt werden. Somit bildet die hier dargestellte Ausführungsform beide Alternativen für ein Anschlusssystem mit feststehender Gewindemutter und drehbeweglicher Überwurfmutter, wobei letztere in wenigstens einem weiteren Herstellungsschritt erzeugt und angeordnet werden muss.

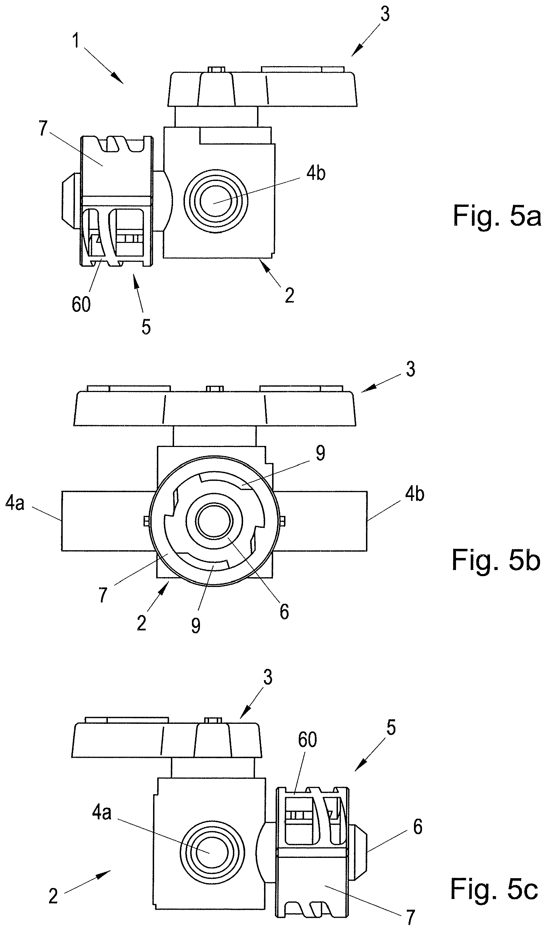

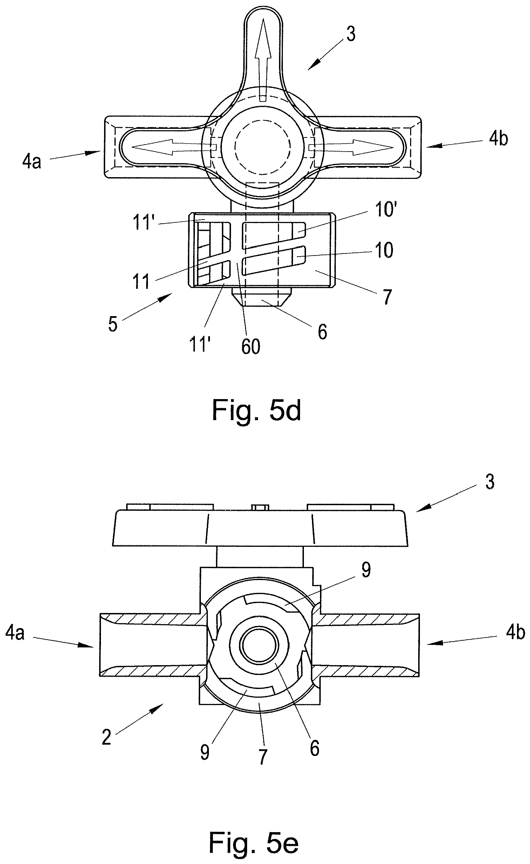

In den Figuren 5a bis 5e ist eine weitere Ausführungsform der vorliegenden Erfindung gezeigt. So zeigt die Figur 5a eine Seitenansicht von rechts, die Figur 5b die Frontansicht, die Figur 5c die Seitenansicht von links und die Figur 5d die Ansicht von oben. Figur 5c ist eine partielle Schnittdarstellung durch die Anschlussstellen 4a und 4b und den Bereich der Gewindemutter mit der Anschlussstelle 5. In dieser Ausführungsform sind die radial umlaufenden Stege 11 bzw. 11' mittels Verstärkungsstreben 60 ergänzt. Diese Verstärkungsstreben, welche im vorliegenden Beispiel parallel zur Haupterstreckungsrichtung der Anschlussstelle 5 angeordnet sind, dienen u.a. dazu, ein Durchbrechen des Gewindes beim Einschrauben eines weiblichen Konnektors zu verhindern. So werden die weiblichen Konnektoren bzw. Anschlussstücke meist mit umlaufenden oder mit zwei Segmenten am Gewinde angeboten. Vor allem bei dem zweigängigen Gewinde ist die Belastung am Gewindeabschnitt der Gewindemutter erhöht und es musste u.a. immer wieder ein Durchbrechen des Gewindes festgestellt werden. Die in diesem Ausführungsbeispiel dargestellten Verstärkungsstreben können dieses Durchbrechen verhindern und bieten somit auch die Möglichkeit, weibliche Konnektoren / Anschlüsse aus verschieden Kunststoffen (wie zum Beispiel duro- und thermoplastische Kunststoff und insbesondere Polyphenylensulfid, Polypropylen, Poly-1-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, Kombinationen hiervon und dergleichen ) einzusetzen. In den Figuren 5a bis 5e wurden ferner Bauteile die im wesentlichen den Ausführungsbeispielen der vorstehenden Ausführungsformen entsprechen mit den gleichen Bezugszeichen versehen.

### Bezugszeichenliste:

- 1: Dreiwegehahn
- 2: Gehäuse
- 3: Küken
- 4a, 4b, 5: Anschlussstellen
- 6, 30: Männlicher Verbindungskonus
- 7: Gewindemutter
- 9: Innengewinde
- 10: Durchbrechungen
- 11, 11': Stege
- 12, 13: Gewindegänge
- 19: Freiraum
- 30: Verbindungskonus
- 31: Nut
- 32: Konische Abstufung
- 33: Verdickung
- 40, 40': Werkzeugteile
- 41, 42, 43: Öffnungen
- 44: Umlaufende Nut
- 45: Anspritzpunkt
- 51, 52, 53: Fluidanschlüsse / Öffnungen
- 53: Anschluss
- 55: Gewindemutter
- 56: Anspritzpunkt
- 60: Verstärkungsstrebe

## Patentansprüche

1. Anschlusssystem für fluiddurchströmte Bauteile in der Medizin und Medizintechnik mit einem männlichen Verbindungskonus (6, 30) und einer wenigstens teilweise den Verbindungskonus (6) umschließenden Gewindemutter (7) mit einem Innengewinde (9), zur verliersicheren Verbindung des männlichen Verbindungskonus (6, 30) mit einer weiblichen Aufnahme eines weiteren fluiddurchströmten Bauteils, **dadurch**
**gekennzeichnet, dass**
die Gewindemutter (7) im axialen Abschnitt ihres Umfangmantels abschnittsweise radial umlaufende Durchbrechungen (10) aufweist und hieran anschließend im Umfangmantel Stege (11, 11') gebildet sind, auf deren Innenseite in Bezug auf den Umfangmantel Abschnitte des Innengewindes (9) angeordnet sind, wobei die Abschnitte des Innengewindes (9) wenigstens die Flanken des Innengewindes sind und die Abschnitte auf die Bereiche der Stege (11, 11') begrenzt sind.

2. Anschlusssystem nach Anspruch 1, **dadurch gekennzeichnet, dass** das Innengewinde (9) zweigängig oder dreigängig ausgeführt ist.

3. Anschlusssystem nach Anspruch 2, **dadurch gekennzeichnet, dass** pro Gewindegang wenigstens zwei Durchbrechungen (10) vorgesehen sind.

4. Anschlusssystem gemäß einem der vorstehenden Ansprüche, **dadurch**
**gekennzeichnet, dass**
sich die radial umlaufenden Durchbrechungen (10) und/oder Abschnitte des Innengewindes (9) in Summe über wenigstens 1/3, vorzugsweise über wenigstens 2/3 des Umfangs erstrecken.

5. Anschlusssystem gemäß einem der vorstehenden Ansprüche, **dadurch**
**gekennzeichnet, dass**
die sich im Umfangsmantel erstreckenden Stege (11) im Bereich der angrenzenden Durchbrechungen (10) Verstärkungsstreben (60) aufweisen.

6. Anschlusssystem gemäß Anspruch 5, **dadurch gekennzeichnet, dass** die Verstärkungsstreben (60) in einem stumpfen Winkel zu den Stegen (11) angeordnet sind.

7. Anschlusssystem gemäß einem der vorstehenden Ansprüche, **dadurch**
**gekennzeichnet, dass**
der Außendurchmesser des Innengewindes (9) größer ist als 7,83 mm und kleiner 20 mm, insbesondere größer ist als 8 mm und kleiner 10 mm und insbesondere bei ungefähr 9 mm liegt und/oder die Steighöhe des Innengewindes größer als 1,3 mm und kleiner 5 mm, insbesondere größer 1,5 mm und kleiner 3 mm und insbesondere ungefähr 1,8 mm bis 2,5 mm ist.

8. Anschlusssystem gemäß einem der vorstehenden Ansprüche, **dadurch**
**gekennzeichnet, dass**
wenigstens Teile des Anschlusssystems aus einem Material hergestellt sind, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, Kombinationen hiervon und dergleichen umfasst.

9. Anschlusssystem gemäß einem der vorstehenden Ansprüche, **dadurch**
**gekennzeichnet, dass**
die Gewindemutter (7) drehfest mit dem Verbindungskonus (6) verbunden ist und insbesondere wenigstens mit dem Verbindungskonus (6) einstückig ausgebildet ist.

10. Anschlusssystem gemäß einem der vorstehenden Ansprüche, **dadurch**
**gekennzeichnet, dass**
die Gewindemutter als Überwurfmutter ausgebildet und vorzugsweise drehbeweglich mit dem Verbindungskonus (30) verbunden ist.

11. Spritzgussverfahren zur Herstellung eines Anschlusssystems gemäß einem der vorstehenden Ansprüche 1 bis 10 mit den Schritten:
- Schließen der Werkzeugform zur Bildung wenigstens des Verbindungskonus (6) und/oder der Gewindemutter (7);
- Einfahren eines Schiebers zur Bildung von Strömungswegen und Freiräumen des Bauteils;
- Anspritzen des Kunststoffes;
- Abkühlen des Kunststoffes in der Werkzeugform;
- Ausfahren der Schieber und Öffnen der Werkzeugform.

12. Spritzgussverfahren zur Herstellung eines Anschlusssystems gemäß Anspruch 11, **dadurch gekennzeichnet, dass**
nach dem Ausfahren der Schieber und Öffnen der Werkzeugform das Spritzgussteil entformt wird.

13. Verwendung der Anschlusssysteme gemäß einem der Ansprüche 1 bis 10 für fluiddurchströmte Bauteile in der Medizin und der Medizintechnik, insbesondere für Infusions- oder Transfusionsschläuche, Y-Verbinder, Mehrwegehähne, Mehrfachverteiler, Injektionsequipment wie Nadeln, Zugänge oder ähnliches und Kombinationen hiervon.

## Claims

1. A connection system for components through which fluid flows in medicine and medical equipment, comprising a male connection cone (6, 30) and a threaded nut (7) at least partially surrounding the connection cone (6) and having an internal thread (9), for captively connecting the male connection cone (6, 30) to a female receptacle of an additional component through which fluid flows,
**characterised in that** the threaded nut (7) has radially circumferential through-holes (10) at least in some sections in the axial section of the circumferential jacket of the threaded nut and webs (11, 11') are formed adjacent to the through-holes in the circumferential jacket, sections of the internal thread (9) being arranged on the inside of said webs in relation to the circumferential jacket, wherein the sections of the internal thread (9) are at least the flanks of the internal thread, and the sections are limited to the regions of the webs (11, 11').

2. The connection system according to claim 1,
**characterised in that** the internal thread (9) is a double thread or a triple thread.

3. The connection system according to claim 2,
**characterised in that** for each screw thread at least two through-holes (10) are provided.

4. The connection system according to any one of the preceding claims,
**characterised in that** the radially circumferential through-holes (10) and/or sections of the internal thread (9) extend over at least 1/3, preferably over at least 2/3, of the circumference.

5. The connection system according to any one of the preceding claims,
**characterised in that** the webs (11), which extend in the circumferential jacket, comprise reinforcement braces (60) in the region of the adjoining through-holes (10).

6. The connection system according to claim 5,
**characterised in that** the reinforcement braces (60) are arranged at an obtuse angle to the webs (11).

7. The connection system according to any one of the preceding claims,
**characterised in that** the external diameter of the internal thread (9) is greater than 7.83 mm and smaller than 20 mm, in particular greater than 8 mm and smaller than 10 mm, and is, in particular, approximately 9 mm, and/or the pitch height of the internal thread is greater than 1.3 mm and smaller than 5 mm, in particular greater than 1.5 mm and smaller than 3 mm, and is, in particular, approximately 1.8 mm to 2.5 mm.

8. The connection system according to any one of the preceding claims,
**characterised in that** at least some parts of the connection system are made from a material selected from a group comprising duroplastics and thermoplastics and in particular polyphenylene sulfide, polypropylene, poly-1-butene, polyvinyl chloride, polyvinylidene chloride, polymethyl metaacrylate, polymethyl methacrylate, polyacrylonitrile, polystyrene, polysulfone, polyacetal, polyvinyl alcohol, polyvinyl acetate, ethylene vinyl acetate (EVA), mixtures comprising ethylene vinyl acetate and polyethylene, ethylene vinylacetate copolymer (EVAC) for example Elvax or Evatane, ionomers, fluorocarbon plastic, polyethylene, polyamide in particular a partially aromatic polyamide, polycarbonate, polyester, polyphenylene oxide, polysulfone, polyvinyl acetal, polyurethane, and chlorinated polyether, cellulose nitrate, cellulose acetate, cellulose ether, phenolic resin, urea resin, thiourea resin, melamine resin, alkyl resin, allyl resin, silicon, polyimide, polybenzimidazole, epoxy resin, casein plastic, crosslinked polyurethane, unsaturated polyester resin, antimicrobial or antiseptic material such as for example highly-porous silver, silver manufactured without ions, silver alloys and in particular microsilver, metal-ions-releasing compounds, materials free of bisphenol A for example Tritan, Terlux, acrylonitrile-butadiene-styrene, combinations thereof and the like.

9. The connection system according to any one of the preceding claims,
**characterised in that** the threaded nut (7) is non-rotatably connected to the connection cone (6), and in particular formed in one piece at least with the connection cone (6).

10. The connection system according to any one of the preceding claims,
**characterised in that** the threaded nut is designed as a union nut and is preferably rotatably connected to the connection cone (30).

11. An injection moulding method for the manufacture of a connection system according to any one of the preceding claims 1 to 10 with the steps of:
- the closing of the mould to form at least the connection cone (6) and/or the threaded nut (7);
- the retraction of a slide gate to form flow paths and spaces of the component;
- the injection of the plastic material;
- the cooling of the plastic material in the mould;
- the extension of the slide gates and the opening of the mould.

12. The injection moulding method for the manufacture of a connection system according to claim 11,
**characterised in that** after the extension of the slide gates and the opening of the mould the injection moulding component is demoulded.

13. The use of the connection systems according to any one of claims 1 to 10 for components through which fluid flows in medicine and medical equipment, in particular for infusion tubes or transfusion tubes, Y-connectors, multiway valves, multiple distributors, injection equipment such as syringes, access devices or similar, as well as combinations thereof.

## Revendications

1. Système de raccordement pour des composants traversés par du fluide dans la médecine et dans la technique médicale, avec un cône de liaison mâle (6, 30) et un écrou fileté (7) entourant au moins en partie le cône de liaison (6) avec un taraudage (9), en vue de la liaison sûre contre la perte du cône de liaison mâle (6, 30) avec un réceptacle femelle d'un autre composant traversé par du fluide, **caractérisé en ce que**
l'écrou fileté (7) présente dans la section axiale de son enveloppe périphérique des percées (10) périphériques radialement par sections, et de manière adjacente à celles-ci, des traverses (11, 11') sont constituées dans l'enveloppe périphérique, sur le côté intérieur desquelles sont disposées par rapport à l'enveloppe périphérique des sections du taraudage (9), les sections du taraudage (9) étant au moins les flancs du taraudage et les sections sur les zones des traverses (11, 11') étant limitées.

2. Système de raccordement selon la revendication 1, **caractérisé en ce que** le taraudage (9) est conçu à deux ou à trois filets.

3. Système de raccordement selon la revendication 2, **caractérisé en ce que** par pas de filet, au moins deux percées (10) sont prévues.

4. Système de raccordement selon une quelconque des revendications précédentes, **caractérisé en ce que**
les percées périphériques radialement (10) et/ou des sections du taraudage (9) s'étendent au total sur au moins 1/3, de préférence sur au moins 2/3 de la périphérie.

5. Système de raccordement selon une quelconque des revendications précédentes, **caractérisé en ce que**
les traverses (11) s'étendant dans l'enveloppe périphérique présentent dans la zone des percées (10) adjacentes des montants de renforcement (60).

6. Système de raccordement selon la revendication 5, **caractérisé en ce que**
Les montants de renforcement (60) sont disposés en angle obtus par rapport aux traverses (11).

7. Système de raccordement selon une quelconque des revendications précédentes, **caractérisé en ce que**
le diamètre extérieur du taraudage (9) est supérieur à 7,83 mm et inférieur à 20 mm, est notamment supérieur à 8 mm et inférieur à 10 mm et se trouve notamment à environ 9 mm et/ou la hauteur de montée du taraudage est supérieure à 1,3 mm et inférieure à 5 mm, notamment supérieure à 1,5 mm et inférieure à 3 mm et est notamment d'environ 1,8 mm à 2,5 mm.

8. Système de raccordement selon une quelconque des revendications précédentes, **caractérisé en ce que**
au moins des parties du système de raccordement sont fabriquées en un matériau, qui est sélectionné à partir d'un groupe qui se compose du duroplastique et du thermoplastique et notamment du polysulfure de phénylène, du polypropylène, du poly-1-butylène, du chlorure de polyvinyle, du chlorure de polyvinylidène, du méta-acrylate de polyméthyle, du polyméthacrylate de méthyle, du polyacrylonitrile, du polystyrène, du polysulfone, du polyacétal, de l'alcool polyvinylique, de l'acétal polyvinylique, de l'éthylène-vinylacétate (EVA), des mélanges d'éthylène-vinylacétate et de polyéthylène, du copolymère éthylène-acétate de vinyle (EVAC) comme par exemple de Elvax ou Evatane, des ionomères, du plastique fluoré, du polyéthylène, du polyamide, notamment un polyamide aromatique partiel, du polycarbonate, du polyester, du polyphénylène oxyde, du polysulfone, de l'acétal polyvinylique, du polyuréthane, et du polyéther chloré, du nitrate de cellulose, de l'acétate de cellulose, de l'éther de cellulose, de la résine phénolique, de la résine d'urée, de la résine thio-urée, de la résine de mélamine, de la résine d'alcoyle, de la résine allylique, de la silicone, du polyimide, du polybenzimidazole, de la résine époxy, du plastique à la caséine, du polyuréthane réticulé, de la résine polyester non saturée, des matériaux antimicrobiens ou antiseptiques, comme par exemple de l'argent hautement poreux, de l'argent fabriqué sans ions, des liaisons d'argent, et notamment du nano-argent, des liaisons dégageant des ions métalliques, des matières sans bisphénol-A, comme par exemple du tritan, du terlux, de l'acrylonitrile-butadiène-styrène, des combinaisons de ceux-ci et des produits similaires.

9. Système de raccordement selon une quelconque des revendications précédentes, **caractérisé en ce que**
l'écrou fileté (7) est relié de manière résistante à la torsion au cône de liaison (6) et notamment constitué au moins en une pièce avec le cône de liaison (6).

10. Système de raccordement selon une quelconque des revendications précédentes, **caractérisé en ce que**
l'écrou fileté est constitué comme écrou-raccord et relié de préférence de manière mobile par rotation avec le cône de liaison (30).

11. Procédé de moulage par injection en vue de la fabrication d'un système de raccordement selon une quelconque des revendications 1 à 10, avec les étapes de :
- Fermeture du moule à outil en vue de la formation d'au moins le cône de liaison (6) et/ou l'écrou fileté (7) ;
- Introduction d'un coulisseau en vue de la formation de chemins d'écoulement et du dégagement du composant ;
- Injection du plastique ;
- Refroidissement du plastique dans le moule à outil ;
- Sortie du coulisseau et ouverture du moule à outil.

12. Procédé de moulage par injection en vue de la fabrication d'un système de raccordement selon la revendication 11, **caractérisé en ce que** après la sortie du coulisseau et l'ouverture du moule à outil, la pièce de moulage par injection est démoulée.

13. Utilisation des systèmes de raccordement selon une quelconque des revendications 1 à 10 pour des composants traversés par du fluide dans la médecine et dans la technique médicale, notamment pour des tuyaux flexibles de perfusion ou de transfusion, des connecteurs en Y, des clapets sélecteurs, des distributeurs multiplex, de l'équipement d'injection tel que les aiguilles, les accès ou similaires et des combinaisons de ceux-ci.
